# EUROPEAN PATENT SPECIFICATION

(11) **EP 0 575 090 B1**
(45) Date of publication and mention of the grant of the patent: **22.04.1998**
(21) Application number: 93304466.1
(22) Date of filing: 09.06.1993
(51) Int. Cl.: A61F 13/00, A61L 15/28, A61L 15/60

(54) **Product suitable for absorbing wound exudate**
Zur Absorbierung von Wundexsudat geeignetes Produkt
Produit apte à absorber l'exsudat humain

(30) Priority: 10.06.1992 GB 9212303; 31.07.1992 GB 9216285
(43) Date of publication of application: 22.12.1993
(73) Proprietor: JOHNSON & JOHNSON MEDICAL, INC., Arlington, Texas 76014 (US)
(72) Inventor: McCabe, John Patrick, Skipton, North Yorkshire BD23 2TE (GB); Stevens, Peter John, Shipley, West Yorkshire BD18 4HX (GB)
(74) Representative: Fisher, Adrian John

(56) References cited:
- EP-A- 0 171 268
- EP-A- 0 344 913
- DE-A- 1 642 146
- FR-A- 2 402 594
- US-A- 3 095 877

## Description

This invention relates to a product suitable for absorbing fluid from wounds during the healing process.

Various products have been used for absorbing fluids such as wound exudate, but they tend to be fibrous and are therefore prone to adhere to the wound and to leave bits in the wound.

Thus, EP-A-0344913 discloses wound dressings of alginate stable fibres which are non-woven fabrics substantially free from any adhesive binder or of interfusing of fibres at their crossing points. Even when its basis weight is as low as 50 g/m², the resulting wound dressing, when saturated with saline fluids, can be removed by forceps from a wound as a single piece, having little or no residue in the wound.

US 3095877 discloses as a surgical packing a nylon thread on which is spaced a plurality of equally spaced half inch diameter balls, typically made of foam material such as urethane foam or foam rubber.

Such difficulties have been overcome by the use of the absorbent product of this invention. This product comprises bits of alginic acid or a salt thereof as an assemblage capable of being applied to the vicinity of a wound to absorb the wound exudate. The form in which the bits are assembled is a string on which the bits are carried directly or perforated bags interconnected by a string in which the bits are housed, which bags are made of a substance of maximum thickness 1 mm which is substantially non-adherent to a wound.

Bits can take various forms. Thus, they can be granules, pellets, spheres or in one specific form beads, i.e. spheres having a hole therethrough, so that they can be strung together. In general, by bit we mean any small discrete quantity which need not be regular in shape and this quantity is usually less than 0.1 g in weight.

Alginic acid or a salt thereof (hereinafter referred to as "alginate") is extracted from seaweed and consists of linear polysaccharides in which the monomeric units are mannuronic acid and gluronic acid. The alginate which is used may, for example, be calcium alginate, zinc alginate, sodium alginate, barium alginate, ammonium alginate or mixtures thereof. Calcium alginate is particularly preferred and may be prepared by an ion exchange reaction between sodium alginate and calcium chloride.

The alginate may however be in the form of a gel, generally from 0.01 to 2.0 mm in thickness. Such gels may suitably be formed by the controlled introduction of a suitable cation (e.g. calcium) into a solution of a water soluble alginate such as sodium alginate, preferably in the presence of a pH modifier such as glucono delta lactone. The alginate concentration may be, for example, from 2 % to 20 % by weight and the final cation concentration may suitably be from 0.2 % to 10 % by weight. The resultant gel will typically contain from 30 % to 99 % by weight of water, and it may then be partially or completely dried if desired, e.g. to a water content of from 15 % to 50 %, and more preferably from 20 % to 40 % by weight.

The assemblage of bits can be of various constructions. Thus, in one form the bits of alginate are housed in a perforated bag interconnected by a string, the bag being made of a thin substance so that in use the exudate from the wound passes through the perforations where it is absorbed by the alginate, for example as pellets, spheres or granules which thereby swell when absorbing the exudate.

The assemblage comprises a string along which beads of alginate are threaded. A suitable length of the string is then placed adjacent to the wound and when the exudate is absorbed by the beads they swell.

In all cases the assemblage is preferably sterile packaged.

When the alginate is housed in a perforated bag the substance from which the bag is made should be of maximum thickness 1 mm, preferably less than 0.5 mm. The substance is also substantially non-adherent to a wound.

The alginate is housed in a series of interconnected perforated bags.

The bags are preferably made of a film, i.e. any suitable material of thickness less than 300 micrometres, for example from 20 to 100 micrometres thick, and preferably from 30 to 70 micrometres thick, e.g. about 60 micrometres.

Each bag which contains alginate is perforated. These perforations should be of sufficient size to allow wound exudate to penetrate, but not so large that substantial portions of the alginate can drop out of the bag. Preferably the alginate is used in the form of small dry spheres, and particularly spheres having a diameter of 0.5 mm to 1.5 mm, for example 1 mm. By "sphere" we do not mean that it has to be a geometrically perfect sphere and could include granules if not too irregular.

The perforations in the bags should have dimensions less than the diameter of such spheres. Typical perforations are squares with sides of 0.5 to 1.00 mm length, e.g. about 0.8 mm.

The amount of alginate in each bag can vary but amounts of from about 1.5 mg to 100 mg per bag are suitable. When the alginate is in the form of spheres each bag preferably has from 1 to 50 spheres, especially from 28 to 32 spheres, e.g. 30 spheres.

The shape of the bags can vary but preferred are substantially rectangular or square bags, preferably of 1 to 5 cm width and 1 to 10 cm length, especially with a width 2 to 3 cm, and length 3 to 4 cm. The depth is preferably about 0.1 cm to 0.4 cm when the alginate is dry.

Alternatively cylindrical bags can be used of length preferably from 1 to 10 cm, especially from 3 to 4 cm. As another alternative, the bags can be for example elliptical cylinders. The diameter of cylindrical bags (or the maximum diameter if the bags are of elliptical section) is preferably from 1 to 5 cm, and more preferably from 2 to 3 cm.

Preferably the series of bags comprises a series of contiguous bags, but this is not essential. The bags are preferably joined to each other side by side, so that the series or line of bags resembles one long flat ribbon. This ribbon is preferably 5 to 30 cm in length and 0.5 to 2.5 cm width.

The bags are connected with a string, thread or cord or similar line running substantially along the length of bags and such a line, e.g. string, thread or cord, is essential when the bags are adjacent to one another but not contiguous with one another. In this case it connects the bags together. Preferably the line runs through each bag and if for example the bags are a series of end-on cuboids or cylinders, the line can run substantially centrally through each cuboid or cylinder, i.e. centrally throughout the length of the ribbon.

The bag or bags must be made of a substance which is substantially non-adherent to the wound, i.e. substantially hydrophobic. Various plastic substances can be used but the preferred plastics is one comprising ethylene/methyl acrylate copolymer and preferably including low density polyethylene. One preferred form comprises 16 % by weight of ethylene/methyl acrylate copolymer, 83 % by weight low density polyethylene and 1 % process additives.

Other suitable plastics materials include water-impermeable polymers, such as a polyolefin. Polyethylene and polypropylene are representative examples of this class, but polymers of higher olefins may of course be used, as may copolymers of two or more olefins, or copolymers of the olefin and one or more other monomers.

Although fibrous plastics material is not preferred because of the risk of portions adhering to the wound it is possible to use such substances if the fibres are fully bonded to one another and there are no loose fibres.

Examples of such fibrous materials which may be used are polyolefins such as polyethylene, polypropylene and polybutylene homopolymers and copolymers, vinyl polymers such as polyvinylchloride, polyamides such as nylon, and polyesters. Other fibres include rayon and acrylic fibres. In particular one can use polyester fibres having a relatively high melting point of approximately 250°C.

To use this form of the product the series of bags, preferably in the form of a ribbon, is fed into the wound. Whilst present in the wound, the alginate preferably as dried spheres, absorbs fluids such as wound exudate, and the alginate if in the form of spheres will swell to produce spheres of diameters of approximately 1 to 3 mm, e.g. about 3 mm. When all the wound exudate has been absorbed the series of bags, e.g. ribbon, now containing hydrated alginate, will be removed and discarded.

When the assemblage comprises a string along which beads of alginate are threaded, the string can be the same or similar to that described above in connection with the bags. The string can be a thin length of cord, thread, twine, fibre or similar material and may be of natural or synthetic material. Thus it may be a nylon thread.

If the beads are spaced at intervals along the string it is preferred that the diameter of the holes in the beads is such that the beads are not too free to slide along the string so that the beads remain spaced apart. A string or fairly course twine having a fairly rough surface, would also be of assistance in this respect. When the beads are spaced apart it is preferred that the average spacing is about the diameter of the bead, e.g. about 3 mm.

If desired the string of beads may be contained in a housing made of thin perforated material. This housing is preferably cylindrical and which preferably houses substantially the whole string of beads. The perforated material from which the housing is made is preferably that described and exemplified above in connection with the perforated bags. In this case however it is not necessary that the perforations are smaller than the diameter of the beads. As before the preferred material for the housing is a plastics comprising ethylene/methyl acrylate copolymer and low density polyethylene.

In a further embodiment the bits of alginate, e.g, spheres of alginate, could contain active wound healing agents, for example growth factors, collagen, glycosaminoglycans, vitamins, antiseptic agents and enzyme debriders.

The advantages of the product of the invention are that it can be completely removed and it is non-adherent. Prior art products tend to be fibrous and are therefore prone to adherence and to leave bits of fibre in the wound.

Specific forms of the invention are described with reference to the drawings in which:-
Fig. 1 shows one form of the invention as a section of a ribbon along line I-I of Fig. 2;
Fig. 2 is a cross-sectional view of the ribbon along line II-II of Fig. 1;
Fig. 3 shows another form of the invention; and
Fig. 4 shows a modification of the invention shown in Fig. 3.

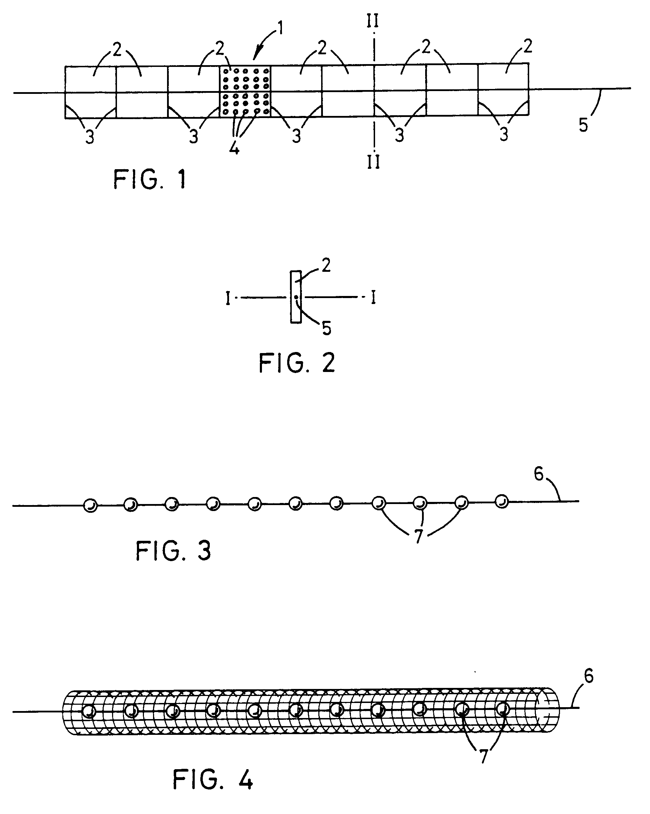

Referring to Figures 1 and 2, 1 indicates a series of rectangular contiguous bags 2 sealed at edges 3. Each bag is made of plastics film comprising ethylene methyl/acrylate copolymer and low density polyethylene and each bag is perforated with perforations 4 as shown on one of the bags.

Each bag contains approximately 56 mg of calcium alginate spheres of diameter less than or approximately equal to 1 mm when dry. A string 5 runs through each of the bags to facilitate entry and removal of the ribbon from the wound. When the spheres absorb exudate they swell to a diameter of about 3 mm.

Referring to Fig. 3 beads of alginate 7 of approximate diameter 3 mm are threaded onto a string 6 at intervals of approximately 4 mm.

Referring to Fig. 4 the string of beads as shown in Fig. 3 is housed in a perforated cylinder made of the same material as used to make the bags shown in Fig. 1 and 2. The perforations are squares of sides approximately 2 mm.

When placed adjacent to a wound the beads of the string of Fig. 3 or of the housed string of Fig. 4 swell as they absorb the exudate.

## Claims

1. A product suitable for absorbing wound exudate comprising bits of alginic acid or a salt thereof, said bits being housed in perforated bags interconnected by a string, which bags are made of a substance of maximum thickness 1mm which is substantially non-adherent to a wound or said bits being less than 0.1 g in weight being carried directly on a string.

2. A product according to claim 1 wherein the alginic acid salt is calcium alginate.

3. A product according to either of claims 1 and 2 wherein the bags are substantially square or rectangular in shape.

4. A product according to any one of the preceding claims wherein the thin substance of maximum thickness 1mm comprises an ethylene/methyl acrylate copolymer.

5. A product according to any one of the preceding claims wherein the alginate is in the form of spheres.

6. A product according to either of claims 1 and 2 wherein beads of alginic acid or salt thereof are threaded along the string.

7. A product according to claim 6 wherein the string of beads is contained in a housing made of thin perforated material.

8. A product according to any one of the preceding claims wherein the thin perforated bags are made of a material comprising ethylene/methyl acrylate copolymer and low density polyethylene.

9. A product according to any one of the preceding claims wherein the bits of alginic acid or salt thereof contain active wound healing agent.

## Patentansprüche

1. Ein zur Absorbierung von Wundexsudat geeignetes Produkt, das Stückchen aus Alginsäure oder einem Salz derselben umfaßt, wobei besagte Stückchen in perforierten Beuteln untergebracht sind, die durch eine Schnur miteinander verbunden sind, wobei die Beutel aus einer Substanz mit einer maximalen Dicke von 1 mm hergestellt sind, die im wesentlichen nicht-anhaftend an einer Wunde ist, oder besagte Stückchen, die ein geringeres Gewicht als 0,1 g aufweisen, direkt auf einer Schnur gehalten werden.

2. Ein Produkt nach Anspruch 1, wobei das Alginsäuresalz Calciumalginat ist.

3. Ein Produkt nach einem der Ansprüche 1 und 2, wobei die Beutel im wesentlichen von quadratischer oder rechteckiger Form sind.

4. Ein Produkt nach einem der vorangehenden Ansprüche, wobei die dünne Substanz mit der maximalen Dicke von 1 mm ein Ethylen/Methylacrylat-Copolymer umfaßt.

5. Ein Produkt nach einem der vorangehenden Ansprüche, wobei das Alginat in der Form von Kügelchen vorliegt.

6. Ein Produkt nach einem der Ansprüche 1 bis 2, wobei die Perlen aus Alginsäure oder Salz derselben auf der Schnur aufgereiht sind.

7. Ein Produkt nach Anspruch 6, wobei die Perlenschnur in einer Umhüllung enthalten ist, die aus einem dünnen perforierten Material hergestellt ist.

8. Ein Produkt nach einem der vorangehenden Ansprüche, wobei die dünnen perforierten Beutel aus einem Material hergestellt sind, das Ethylen/Methylacrylat-Copolymer und Polyethylen niedriger Dichte umfaßt.

9. Ein Produkt nach einem der vorangehenden Ansprüche, wobei die Stückchen aus Alginsäure oder Salz derselben aktives Wundheilungsmittel enthalten.

## Revendications

1. Produit approprié pour absorber l'exsudat des plaies comprenant des fragments d'acide alginique ou de son sel, lesdits fragments étant logés dans des sachets perforés reliés par un cordon, ces sachets étant en une substance d'une épaisseur maximale de 1 mm, sensiblement non adhérente à une plaie ou lesdits fragments étant inférieurs à 0,1 g en poids étant portés directement sur un cordon.

2. Produit selon la revendication 1, dans lequel le sel d'acide alginique est l'alginate de calcium.

3. Produit selon la revendication 1 ou 2, dans lequel les sachets sont de forme sensiblement carrée ou rectangulaire.

4. Produit selon l'une quelconque des revendications précédentes, dans lequel la substance mince d'une épaisseur maximale de 1 mm comprend un copolymère éthylène/acrylate de méthyle.

5. Produit selon l'une quelconque des revendications précédentes, dans lequel l'alginate est sous forme de sphères.

6. Produit selon la revendication 1 ou 2, dans lequel les perles d'acide alginique ou de son sel sont enfilées le long d'un cordon.

7. Produit selon la revendication 6, dans lequel le cordon de perles est contenu dans un logement en matière mince perforée.

8. Produit selon l'une quelconque des revendications précédentes, dans lequel les sachets perforés minces sont en une matière comprenant un copolymère éthylène/acrylate de méthyle et un polyéthylène à basse densité.

9. Produit selon l'une quelconque des revendications précédentes, dans lequel les fragments d'acide alginique ou de son sel contiennent un agent actif de cicatrisation.
